# EUROPEAN PATENT SPECIFICATION

(11) **EP 1 532 130 B1**
(45) Date of publication and mention of the grant of the patent: **04.11.2009**
(21) Application number: 02807711.3
(22) Date of filing: 29.08.2002
(51) Int. Cl.: C07D 307/88

(54) **PROCESS FOR THE PRODUCTION OF AN IMMUNOSUPPRESSANT**
VERFAHREN ZUR HERSTELLUNG EINES IMMUNOSUPPRESIVUMS
PROCEDE DE PRODUCTION D'UN IMMUNOSUPPRESSEUR

(43) Date of publication of application: 25.05.2005
(73) Proprietor: Biocon Limited, Bangalore 561 229 (IN)
(72) Inventor: POORNAPRAJNA, Acharya, Bangalore 561229 Karnataka (IN); SREENILAYAM, Gopeekrishnan, Bangalore 561229 Karnataka (IN); GANESH, Sambasivam, Bangalore 561229 Karnataka (IN)
(74) Representative: Freyria Fava, Cristina
(86) International application number: PCT/IN2002/000178
(87) International publication number: WO 2004/020426

(56) References cited:
- ZA-A- 6 804 959
- GRETY RIHS ET AL.: 'Sodium Mycophenolate' ACTA CRYSTALLOGRAPHICA SECTION C vol. C56, no. 4, April 2000, pages 432 - 433, XP008052367

## Description

### FIELD OF INVENTION

The present invention provides a method for producing the sodium salt of the compound of Formula I.

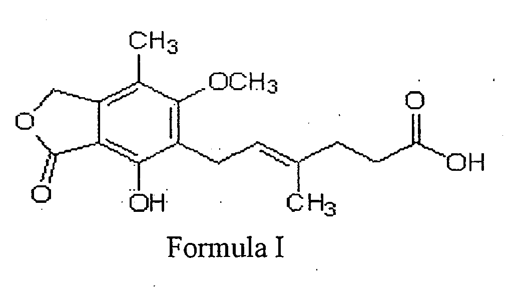

### BACKGROUND OF THE INVENTION

Mycophenolic acid is an immunosuppressive agent that inhibits *de novo* purine nucleotide synthesis via inhibition of IMP dehydrogenase and prevents the formation of XMP and GMP.

Sodium salt of Mycophenolic acid or ERL 080 has been widely discussed in available patent and non-patent literature in treatment of diseases and in transplantation.

Use of Sodium Salt of Mycophenolalic acid in treatment of hyperuricaemia has been reported in U.S. patent no. 3,705,946. US Patent Nos. 6,025,391 and 6,172,107 describes an enteric coating composition. In US Patent No. 6025391 the enteric coating composition contains HPMC phthalate and triacetin prepared for capsules containing monosodium mycophenolate; adapted to release mycophenolate in the upper part of the intestinal tract.

South African Patent no. 6814951 relates to the Sodium salt of MPA.

Tolerability profile of sodium mycophenolate and mycophenolate mofetil with and without cyclosporin has been discussed in Toxicology 157(2001) 207-215.

Che journal Acta Crystallographica, Section C: Crystal Structure Communications (2000), C56(4), 432-433 discusses Crystal stucture of Sodium mycophenolate.

### SUMMARY OF THE INVENTION

The instant invention reveals a process for manufacture of Sodium salt of the compound of formula I, which comprise reacting the compound of formula I with sodium salt of C₂ to C₁₀ carboxylic acid

### DETAILED DESCRIPTION OF THE INVENTION

The instant invention describes a Process of manufacturing Sodium salt of the compound of formula I, 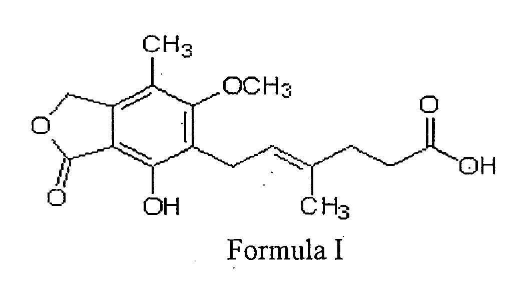 by reacting the compound of formula I with a sodium salt of C₂ to C₁₀ carboxylic acid.

Alternately the compound of formula I may be converted to its ammonium or dibenzyl amine form by reacting compound of formula I with a sodium salt of C₂ to C₁₀ carboxylic acid

The compound of formula I is converted to its ammonium salt by treating with ammonia.

The dibenzyl amine salt of the compound of formula I is prepared by reacting with dibenzyl amine.

The sodium salt of C₂ to C₁₀ carboxylic acid is selected from sodium acetate, sodium 2-ethyl hexanoate or sodium caprylate.

The compound of formula I is converted to its ammonium salt by reacting with ammonia. The ammonium salt of formula I is reacted with sodium acetate or sodium 2-ethyl hexanoate or Sodium caprylate to get the sodium salt of the compound of formula I.

The following Examples further illustrate the invention, it being understood that the invention is not intended to be limited by the details disclosed therein.

### EXAMPLE 1

10g of sodium acetate is dissolved in 55 ml of methanol. To this 74g of mycophenolic acid was added and stirred for half an hour at RT. The contents were chilled to 10°C and filtered. The solid are washed with 50 ml acetone, dried under vacuum at 40 to 50°C. A final yield 90%( 70g) was observed.

### EXAMPLE 2

To a slurry of mycophenolic acid (25g) in methanol, dicyclohexyl amine was added and stirred at RT. The precipitated solid was then treated with aqueous sodium acetate solution under stirring RT. The reation mixture was cooled to 10 °C and the precipitated solid was filtered and dried.

### EXAMPLE 3

25g of sodium 2-ethylcaprylate is dissolved in 175 ml of methanol. To this 250g of mycophenolic acid was added and stirred for half an hour at RT. The contents were chilled to 10°C and filtered. The solid are washed with 50 ml acetone, dried under vacuum at 40 to 50°C. A final yield 90%( 22g) was observed.

### EXAMPLE 4

25g of sodium 2-ethylhexanoate is dissolved in 175 ml of ethyl acetate. To this 250g of mycophenolic acid was added and stirred for half an hour at RT. The contents were chilled to 10°C and filtered. The solid are washed with 50 ml acetone, dried under vacuum at 40 to 50°C. A final yield 90%( 22g) was observed.

### EXAMPLE 5

100g of mycophenolic acid was taken in 175 ml of methanol and was stirred for half an hour at RT. Ammonia gas was bubbled for 30 min followed by addition of aqueous sodium acetate. The contents were chilled to 10°C and filtered. The solid are washed with 50 ml acetone, dried under vacuum at 40 to 50°C. A final yield 90% ( 95g) was observed.

## Claims

1. A Process of manufacturing sodium salt of the compound of formula I comprising, 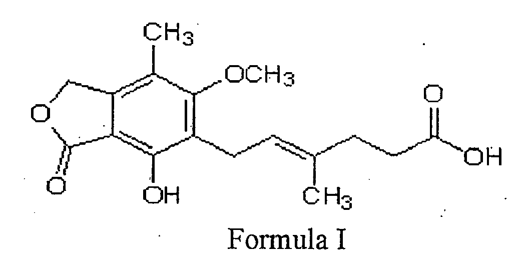 reacting the compound of formula I with a sodium salt of C₂ to C₁₀ carboxylic acid.

2. A process according to claim 1, wherein the compound of formula I is converted to an ammonium salt or a dibenzyl amine salt before converting it to the sodium salt.

3. A process according to claim 2, wherein the compound of formula I is converted to its ammonium salt by treating with ammonia.

4. A process according to claim 2, wherein the dibenzyl amine salt of the compound of formula I is obtained by reacting with dibenzyl amine.

5. A process according to claim 1, in which sodium salt of C₂ to C₁₀ carboxylic acid is selected from sodium acetate, sodium 2-ethyl hexanoate or sodium caprylate.

## Patentansprüche

1. Verfahren zur Herstellung des Natriumsalzes der Verbindung der Formel I umfassend 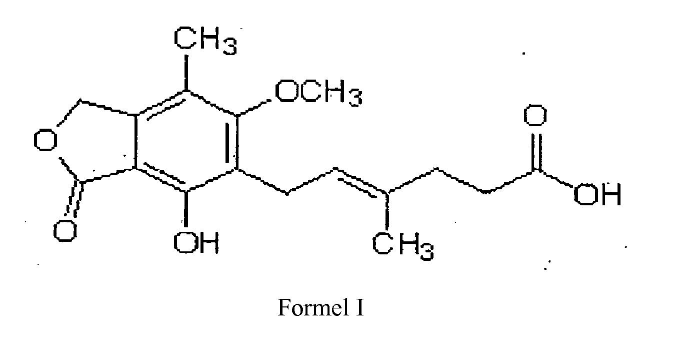 Umsetzen der Verbindung der Formel I mit einem Natriumsalz einer C₂-bis C₁₀-Carbonsäure.

2. Verfahren gemäß Anspruch 1, wobei die Verbindung der Formel I zu einem Ammoniumsalz oder einem Dibenzylaminsalz vor der Umsetzung zu dem Natriumsalz umgesetzt wird.

3. Verfahren gemäß Anspruch 2, wobei die Verbindung gemäß Formel I zu einem Ammoniumsalz umgesetzt wird, indem sie mit Ammoniak behandelt wird.

4. Verfahren gemäß Anspruch 2, wobei das Dibenzylaminsalz der Verbindung gemäß Formel I erhalten wird, indem sie mit Dibenzylamin umgesetzt wird.

5. Verfahren gemäß Anspruch 1, wobei das Natriumsalz der C₂- bis C₁₀-Carbonsäure unter Natriumacetat, Natrium-2-ethylhexanoat oder Natriumcaprylat gewählt ist.

## Revendications

1. Un procédé de fabrication d'un sel de sodium du composé de formule I 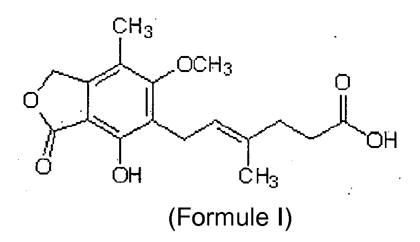 comprenant :
la mise en réaction du composé de formule I avec un sel de sodium d'acide carboxylique en C₂ à C₁₀.

2. Un procédé selon la revendication 1, dans lequel le composé de formule I est converti en un sel d'ammonium ou un sel de dibenzylamine avant de le convertir en le sel de sodium.

3. Un procédé selon la revendication 2, dans lequel le composé de formule I est converti en son sel d'ammonium par traitement avec de l'ammoniac.

4. Un procédé selon la revendication 2, dans lequel le sel de dibenzylamine du composé de formule I est obtenu par mise en réaction avec de la dibenzylamine.

5. Un procédé selon la revendication 1, dans lequel le sel de sodium d'acide carboxylique en C₂ à C₁₀ est choisi parmi l'acétate de sodium, le 2-éthyl hexanoate de sodium ou le caprylate de sodium.
